# EUROPEAN PATENT APPLICATION

(11) **EP 1 774 982 A1**
(43) Date of publication of application: **18.04.2007**
(21) Application number: 05022487.2
(22) Date of filing: 14.10.2005
(51) Int. Cl.: A61L 9/00, A61L 9/04, B60H 3/00

(54) **A phial for a perfuming and/or deodorising device**

(71) Applicant: Altmann, Fritz, 20152 Milan (IT)
(72) Inventor: Altmann, Fritz, 20152 Milan (IT)
(74) Representative: Coloberti, Luigi

(57) **Abstract**

A phial containing a volatile liquid substance for a perfuming and/or deodorising device for vehicles and small environments; the phial (10) comprises a hollow body (13) of plastic material, having an elongated bottom portion (14) for containing the liquid substance (11), an upper cup-shaped portion (18) for collecting any surplus of liquid substance (11) sucked by a wick (12), and an intermediate neck (19) for tightly retaining the wick (12). A closure portion (20) is removably fastened to the cup-shaped portion (18) by a breakable weakened line (23).

## Description

### BACKGROUND OF THE INVENTION

This invention concerns a device for emanating volatile deodorising and/or perfuming substances in the interior compartment of a vehicle, or in small environments.

### STATE OF THE ART

It is known that deodorising-perfuming devices are used in order to emanate a pleasant fragrance inside the interior compartment of vehicles or in small environments, in which unpleasant odours very often develop, due for example to stagnant humidity, cigarette smoke, and the like, which make the environments themselves somewhat unwelcoming.

In particular, in order to make the interior compartment of a vehicle more comfortable, by eliminating any unpleasant odours, use is generally made of deodorising devices which, as a means of diffusion, exploit an air stream flowing from an air ventilating outlet of the heating and air-conditioning system of the interior compartment of the vehicle.

For example, a type of deodorising device is known which comprises a supporting casing of plastic material, designed to hold a container, generally made of glass or plastic, filled with essential oils, in which a special wick facilitates the suction and emanation of the perfuming substance from the container.

This diffuser is clipped onto an air ventilating outlet of a heating and air-conditioning system, by appropriate fastening means which extend from the supporting casing, for example consisting of a clip which is secured to a grid of the aforesaid air ventilating outlet.

Conventionally, the container for the essential oils is provided with a plug member of plastic material, having an axial hole for the insertion of the wick, which can consequently be held in such a position as to protrude from the container, while at the same time remaining partially immersed in the essential oils inside the container.

The wick may for example be made in the form of a stick comprising longitudinal capillary vessels which convey the essential oils from the container to such a height as to be struck by the air stream flowing from the air venting aperture.

The plug is in the form of a funnel shaped member comprising a recovery channel which allows any excess of essential oils sucked by the wick, to flow back into the container.

A deodorising device of this kind however comprises different parts which must be fitted together during manufacturing. This involves high manufacturing costs, due both to the production of the different parts of the device, and to their assembling.

Moreover, this deodorising device has several drawbacks, due to the possibility of accidental spillage of the essential oils, which on contact with fabrics and plastic materials can cause damage of an aesthetical and functional nature.

Furthermore, the device can be easily dismantled from the air ventilating aperture and this constitutes a potential hazard whenever young children are present.

### OBJECTS OF THE INVENTION

The main object of this invention is to provide a phial for containing a perfuming and/or deodorising substance suitable for a deodorizing device, which is structurally simple and which permits a significant reduction in manufacturing costs, compared to the conventional containers used in the perfuming and/or deodorising devices of the above-mentioned kind.

A further object of this invention is to provide a phial or vial for containing a perfuming and/or deodorising substance of the aforementioned kind, which offers a high degree of intrinsic safety degree against spillage of perfuming substances, both in the case of normal use, and in the event of improper handling, for example by small children.

A still further object of the invention is to provide a deodorising and/or perfuming device for vehicles and small environments, comprising a replaceable phial for containing a perfuming and/or deodorising substance, which is structurally simple and inexpensive.

### BRIEF DESCRIPTION OF THE INVENTION

The foregoing can be achieved by means of a phial for containing a liquid perfuming and/or deodorising volatile substance, by means of a wick partially extending into the phial itself,
characterised by comprising an hollow body of plastic material, having a bottom and a peripheral wall defining a cavity for containing the liquid perfuming and/or deodorising substance;
a cup-shaped upper portion, for collecting any excess of liquid substance sucked by the wick; and
an intermediate neck portion for tightly retaining the wick, said neck portion extending between the hollow body and the upper cup-shaped portion of the phial, and a removable closure portion connected to the cup-shaped portion by a breakable weakened line.

### BRIEF DESCRIPTION OF THE DRAWINGS

These and further features of the invention, will be more clearly evident from the following description with reference to the accompanying drawings, in which:
- Fig. 1 shows a front view of a phial for containing a liquid perfuming and/or deodorising substance, according to this invention;
- Fig. 2 shows a side view of the phial of Fig. 1;
- Figs. 3A and 3B show cross-sectional views of possible embodiments of the neck and wick of the phial, along the line 3-3 of Fig. 1;
- Figs. 4 and 5 show the phial of fig. 1 with the closure portion removed and the wick partially or totally inserted into the phial, in two different positions.

### DETAILED DESCRIPTION OF THE UTILITY MODEL

The general features of this phial according to the invention will be more illustrated hereunder by means of an exemplificative embodiment.

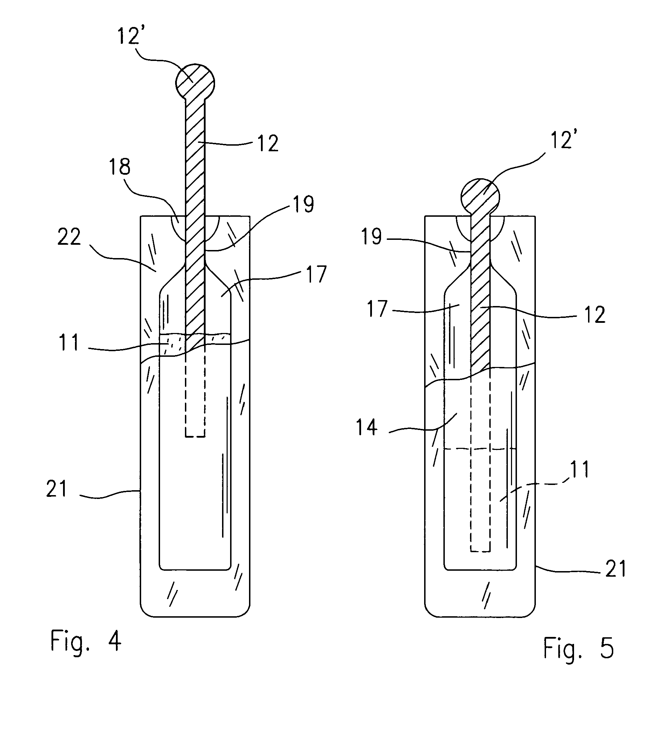

Figures show a phial 10 according to the invention, for containing a liquid perfuming and/or deodorising substance 11, visible at two different levels in figures 4 and 5, capable of being diffused by evaporation by a wick 12 which is inserted and partially extends into the phial 10 once the phial 10 has been opened, as can be seen in figures 4 and 5.

The phial 10 according to this invention can be removably inserted into a supporting casing, not shown, thereby forming a perfuming-deodorising device for vehicles and/or small environments, which can for example be secured or clipped onto an air ventilating aperture of a heating and air-conditioning system of a vehicle, by means of appropriate fastening means.

The phial 10 comprises an elongated hollow body 13 of plastic material, having a bottom 15 and a peripheral wall 16 defining a cavity 17 for containing the liquid perfuming and/or deodorising substance 11.

The body 13 of the phial 10 also has an upper cup-shaped portion 18, for collecting any excess of liquid substance 11 sucked by the wick 12, and an intermediate neck 19 for tightly retaining the wick 12, which extends between the body 13 and the upper cup-shaped portion 18 of the phial.

The upper cup-shaped portion 18, before use, is tightly closed by a closure portion 20, made in one piece with the body 13 of the phial 11, which can be removed by breakage for opening the phial 10 and enabling its use.

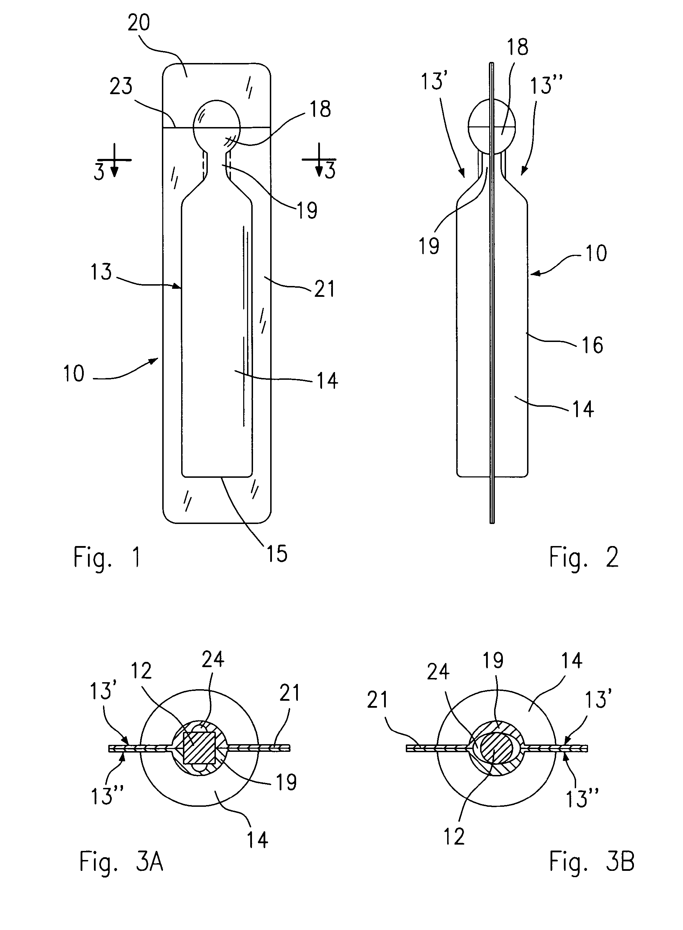

As shown in Fig. 2, the body 13 of the phial 10 is preferentially composed of a first and a second thermoformed half shells 13', 13" sealed together along peripheral edges to form the body 13 of the phial 10.

As an alternative, the body 13 of the phial 10 may be shaped by blowing, or by any other suitable manufacturing process.

The two half shells 13', 13" are respectively provided with peripheral flanges 21 defining flat coupling and fastening surfaces 22 for a reciprocal coupling and fastening the same half shells 13', 13" of the phial 10.

The closure portion 20 is preferentially connected to the upper cup-shaped portion 18, by a weakened breaking line 23.

The intermediate neck 19 for retaining the wick 12, can be provided with at least one longitudinal channel 24, extending between the upper cup-shaped portion 18 and the bottom portion 14 of the phial 10, for the recovery of any excess of perfuming and/or deodorising substance 11 collected by the upper cup-shaped portion 18.

Said channel 24 can, for example, be defined by a slot on the internal surface of the neck 19, or can be defined by opposite facing surfaces of the neck 19 and the wick 12 itself.

The cross-section of the neck 19 has a cross shape which at least partially conforms to the cross-section of the wick 12, so as to prevent any accidental spillage of the liquid substance 11.

In order to improve the diffusion of the perfuming and/or deodorising substance 11, the wick 12, at the upper end protruding from the phial 10, has a widened portion 12' defining a large evaporating surface for the perfuming and/or deodorising substance 11, which may be of any shape, in relation to aesthetical or personalising requirements.

The wick 12 may, for example, be obtained by cutting from cardboard, or by extrusion, having in this latter case longitudinal capillary vessels, to ensure the efficient suction of the liquid substance 11 from the cavity 17 of the phial.

A phial 10 according to this invention is therefore structurally simple and does not involve any assembling operations whatsoever, in that the various functional parts of the phial 10 are made in one single piece, thereby enabling a significant reduction in manufacturing costs.

The phial 10 may be made in different shapes, in relation both to aesthetical requirements, and of functional nature for its insertion into a supporting casing, together with which it constitutes a perfuming and/or deodorising device.

The use of a wick 12 obtained by cutting a sheet of cardboard material, for example of the micro-perforated type, avoids the need to create the recovery channel 24 along the neck 19 of the phial 10, in that it automatically absorbs any excess of liquid substance 11.

In this way, the phial 10 is hermetically sealed, thereby avoiding the risk of accidental spillage of liquid substance 11 in the event of the product being dropped, or improperly handled by small children or by a user.

What has been described and shown with reference to the accompanying drawings, has been given purely by way of example in order to illustrate the general features of the invention, and of one of its preferential embodiments; therefore, other modifications and variations to the phial for containing a perfuming and/or deodorising substance are possible, without thereby departing from the claims.

## Claims

1. A phial (10) suitable for a diffusing device, containing a perfuming and/or deodorising liquid substance (11), to be sucked by a wick (12) partially extending into the phial (10),
**characterised by** comprising a hollow body (13) of plastic material, having an elongated hollow portion (14) comprising a bottom (15) and a peripheral wall (16) defining a cavity (17) for the perfuming and/or deodorising liquid substance (11);
a upper cup-shaped portion (18), for collecting any excess of liquid substance (11) sucked by the wick (12);
an intermediate neck (19) for tightly retaining the wick (12), said neck (19) extending between the hollow portion (14) and the upper cup-shaped portion (18) of the phial (10); and
a closure portion (20) removably connected to the cup-shaped portion (18) of the phial (10).

2. The phial (10) according to claim 1, **characterised in that** said body (13) of plastic material comprises a first and a second thermoformed half shells (13',13") sealingly joined together to form the body (13) of the phial (10).

3. The phial according to claim 2, **characterised in that** said first and second half shells (13',13") are provided respectively with peripheral flanges (21) defining flat coupling surfaces (22).

4. The phial according to claim 1, **characterised in that** the closure portion (20) is fastened to the cup-shaped portion (18), by a weakened breaking line (23).

5. The phial according to claim 1, **characterised in that** the intermediate neck (19) is provided with at least one longitudinal channel (24), extending between the upper cup-shaped portion (18) and the bottom portion (14) of the phial (10).

6. The phial according to claim 5, **characterised in that** said channel (24) is defined by a slot on the internal surface of the neck (19).

7. The phial according to claim 5, **characterised in that** said channel (24) is provided into opposite facing surfaces of the neck (19) and the wick (12).

8. The phial according to claim 1, **characterised in that** the hollow body (13) of the phial (10) is performed by blowing.

9. The phial according to claim 1, **characterised in that** the cross-section of the neck (19) at least partially conforms to the cross-section of the wick (12).

10. The phial according to claim 1, **characterised in that** the wick (12), is provided with a widened head portion (12') defining a larger evaporating surface.

11. The phial according to claim 1, **characterised in that** the wick (12) is cut from a cardboard sheet.

12. The phial according to claim 1, **characterised in that** the wick (12) is provided by extrusion piece having longitudinal capillary vessels.

13. A perfuming-deodorising device for vehicles and/or small environments, suitable for a phial according to claim 1, **characterised by** comprising a phial supporting case, said phial (10) being removably disposed into said supporting case.
